# EUROPÄISCHE PATENTANMELDUNG

(11) **EP 1 192 962 A1**
(43) Veröffentlichungstag der Anmeldung: **03.04.2002**
(21) Anmeldenummer: 01122850.9
(22) Anmeldetag: 24.09.2001
(51) Int. Cl.: A61M 1/16, A61M 25/04, G01N 21/03, A61M 1/36

(54) **Verfahren und Vorrichtung zur Ultrafiltrationskontrolle bei Blutbehandlungsverfahren**

(30) Priorität: 26.09.2000 DE 10047421
(71) Anmelder: Polaschegg, Hans-Dietrich, Dr.techn., 9231 Köstenberg (AT)
(72) Erfinder: Sodemann, Klaus, Dr. med. (Nephrologe), 77933 Lahr (DE); Polaschegg, Hans-Dietrich, Dr.techn., 9231 Köstenberg (AT)

(57) **Zusammenfassung**

Es wird ein Verfahren und eine Vorrichtung beschrieben, mit deren Hilfe Symptome wie Blutdruckabfälle und Krämpfe bei der Ultrafiltrationsbehandlung mit Hilfe der Hämodialyse oder Hämofiltration verringert werden können. Es wurde erkannt, daß die gemischtvenöse Sauerstoffsättigung vor dem Auftreten solcher Symptome stark abfällt und dieser Abfall eine bessere Vorhersage von Symptomen ermöglicht als eine kontinuierlich Blutdruck- oder Blutvolumenmessung.

Mit Hilfe eines Sauerstoffsensors (270) in der Blutbehandlungseinrichtung wird der Sauerstoffgehalt des venösen Blutes gemessen und damit der Flüssigkeitsentzug bei der Hämodialyse oder Hämofiltration gesteuert. Venöses Blut wird mit einem rechtsatrialen Katheter entnommen und entweder über einen weiteren Katheter zentralvenös oder über eine Fistel in den Blutkreislauf des Patienten zurückgeführt. Der Sauerstoffgehalt kann entweder mit Hilfe eines Sauerstoffsättigungssensors im extrakorporalen Kreislauf (200) oder mit einem Sauerstoffpartialdrucksensor gemessen werden.

## Beschreibung

Die Erfindung betrifft ein Verfahren und eine Einrichtung zur Kontrolle der Ultrafiltration bei der Hämodialyse, Hämofiltration oder Hämodiafiltration. Die genannten Verfahren werden zur akuten und chronischen Behandlung des Nierenversagens herangezogen. Beim Nierenversagen sammelt sich im Körper des Patienten überschüssige Flüssigkeit an, die durch die Behandlung entzogen werden muß. Dabei treten zwei Probleme auf. Erstens muß die Menge des überschüssigen Wassers ermittelt werden. Zweitens muß das Wasser so schonend entzogen werden, daß es dabei nicht zu Nebenwirkungen wie z.B. Blutdruckabfällen oder Krämpfen kommt. Das erste Problem, die Bestimmung des überschüssigen Wassers oder des Trockengewichts wird als Aufgabe des Mediziners betrachtet, der das "Trockengewicht" mit Hilfe seines Sachverstandes festlegt. Die Differenz zwischen dem aktuellen Gewicht des Patienten und dem verschriebenen Trockengewicht ist dann die zu entziehende Wassermenge. Das zweite Problem - der schonende Entzug der überschüssigen Wassermenge - kann in der Regel durch eine lange Behandlungsdauer gelöst werden. Nebenwirkungen treten bei einer Behandlungsdauer von 8 Stunden dreimal pro Woche selten auf. Alternativ kann auch täglich und dafür kürzer behandelt werden.

Eine derart lange Behandlungsdauer bedeutet nicht nur für den Patienten eine Belastung, sondern führt auch zu hohen Kosten im Gesundheitssystem. Man hat daher schon frühzeitig nach Möglichkeiten gesucht, die Ultrafiltrationsbehandlung so zu steuern, daß Nebenwirkungen vermieden werden können, die Behandlungszeit aber auf 4 bis 5 h, 3x pro Woche oder weniger beschränkt werden kann. Der erste Schritt dazu war die Entwicklung von Geräten, die einen kontrollierten Entzug der Flüssigkeit erlauben. Solche Geräte sind heute Stand der Technik. Eine Einrichtung zur volumetrischen Flüssigkeitskontrolle ist z.B. in der deutschen Patentanmeldung 2858205 beschrieben. Mit Hilfe einer solchen Vorrichtung kann nun Flüssigkeit aus dem extrakorporalen Kreislauf und damit aus dem Blut des Patienten über die Dialysator- oder Filtermembran mit einer konstanten, vorgegebenen Rate entzogen werden. Da nun der größte Teil der überschüssigen Flüssigkeit nicht im Blutraum des Patienten sondern im Extrazellulärraum gespeichert ist und der Nachstrom dieser Flüssigkeit in den Blutraum nicht durch ein Gerät beeinflußt werden kann, kommt es bei zu schnellem Flüssigkeitsentzug häufig zu einer so starken Verminderung des Blutvolumens, daß Symptome wie Blutdruckabfälle oder Krämpfe auftreten.

Man hat nun schon sehr früh erkannt, daß diese Symptome mit dem effektiven zirkulierenden Blutvolumen zusammenhängen, hat aber bis heute keine praktikable Methode gefunden um dieses bei jeder Behandlung messen zu können. Hingegen hat man Verfahren entwickelt, die die Veränderung des Blutvolumens messen. Dazu verwendet man Sensoren, die den Hämatokrit, den Hämoglobin oder Gesamtproteingehalt des Blutes messen. Physikalisch erfolgt dies z.B. durch Messung der optischen Dichte, der Dichte, der elektrischen Leitfähigkeit oder der Viskosität. Ein solches Verfahren ist z.B. in der deutschen Patentanmeldung 3827553 beschrieben. Eine Einrichtung zur Messung des Hämatokrits mit Hilfe von optischen Sensoren ist z.B. im US Patent 5499627 beschrieben. Die dort beschriebene Einrichtung verwendet mehrere Wellenlängen um Geometriekonstanten aber auch Nebeneinflüsse zu eliminieren. Zu diesen Nebeneinflüssen gehört die von der Sauerstoffsättigung abhängige Absorption von Hämoglobin. Mit Hilfe einer solchen Einrichtung ist es dann möglich, nicht nur den Hämatokrit sondern gleichzeitig auch die Sauerstoffsättigung des Blutes zu messen. Eine entsprechende Einrichtung wird als "Crit-Line Monitor" von der Fa. In-Line Diagnostics in Utah/USA produziert und vermarktet. Mit Hilfe der Sauerstoffsättigungsmessung im extrakorporalen Kreislauf hat man dann auch klinisch den Einfluß von Schlafapnöen (Atemstillstand) registriert.

Man hat nun versucht die Ultrafiltrationsrate von der Veränderung des Blutvolumens abhängig zu steuern. Bisher hat man aber noch keine allgemeingültige Regel gefunden, die prospektiv mit großer Wahrscheinlichkeit das Auftreten von Symptomen verhindert. Ein Verfahren, das im klinischen Test bei etwa der Hälfte der Patienten die Zahl der Nebenwirkungen deutlich vermindert hat, besteht darin, den Hämatokrit zu registrieren, der bei Auftreten eines Symptoms, z.B. eines Blutdruckabfalls, gemessen wird. Dieser Hämatokrit charakterisiert dann ein bestimmtes, allerdings unbekanntes Blutvolumen, bei dem Symptome auftreten. Bei nachfolgenden Behandlungen wird dann die Ultrafiltration abgeschaltet, bevor dieser Hämatokritwert erreicht wird. Da nun aber der Hämatokrit des Patienten nicht nur vom Grad der Überwässerung abhängt, sondern auch von der Blutbildungsrate, die wiederum durch die Gabe von Erythropoietin, einem die Blutbildung förderndem Hormon, beeinflußt wird, ist die Festlegung eines solchen klinischen Grenzwertes des Hämatokrits stets nur für beschränkte Dauer möglich.

Versuche aus der Geschwindigkeit der Blutdruckveränderung Rückschlüsse auf das bevorstehende Auftreten von Symptomen zu ziehen haben bisher auch keinen Erfolg gebracht. Das Problem besteht u.a. darin, daß die Biutvolumenveränderung bei einer Dialysebehandlung nur einige Prozent bis 28 Prozent im Extremfall beträgt und daß häufig beobachtet wird, daß Symptome nicht beim niedrigsten, während der Behandlung gemessenen Blutvolumen auftreten. Obwohl Einrichtungen zur Messung der Veränderung des Blutvolumens nun seit mehreren Jahren klinisch verfügbar und in Tausenden von Dialysegeräten eingebaut sind, ist eine Verbesserung der Situation nicht abzusehen. Es handelt sich dabei nicht um ein technisches, sondern ein medizinisch-physiologisches Problem.

In der Regel wird für die extrakorporale Blutbehandlung des Nierenversagens eine arterio-venöse Fistel als Blutzugang herangezogen. Aus medizinischen Gründen ist dies aber bei einer zunehmenden Zahl von Patienten nicht mehr möglich. Diese werden dann gewöhnlich mit Hilfe von zentralvenösen Kathetern, deren Spitze im rechten Vorhof plaziert ist, behandelt.

Bei der Dialysebehandlung von Patienten mit zentralvenösen Kathetern als Blutzugang, die mit einem Crit-Line Monitor der Fa. In-Line Diagnostics überwacht wurden, wurde nun überraschenderweise festgestellt, daß die Sauerstoffsättigung des extrakorporalen Blutes unmittelbar vor Auftreten eines Symptoms dramatisch abfiel, obwohl das Blutvolumen keinen auffälligen Abfall zeigte. Daraufhin angestellte Überlegungen führten zu dem Schluß, daß das aus dem rechten Vorhof entnommene Blut infolge der Reduktion des effektiven zirkulierenden Blutvolumens stärker von Sauerstoff ausgeschöpft wird. Auf Grund der Durchmischung mittels einer Blutpumpe ist es wohl repräsentativ für das gemischte venöse Blut aus der oberen und unteren Hohlvene und die darin gemessene Sauerstoffsättigung entspricht annähernd der gemischt-venösen Sättigung. Die gemischt-venöse Sättigung aus dem Mischblut der oberen und unteren Hohlvene ist ein in der Kardiologie und Intensivmedizin bekannter Parameter, der dort allerdings an Blut aus der Pulmonalarterie mit Hilfe spezieller Katheter gemessen wird (kontinuierliche fiberoptische Methode oder intermittierende Blutentnahme und Messung mittels eines Oxymeters).

Die gemischt-venöse Sättigung ist Ausdruck für die Sauerstoffausschöpfung im Gewebe und den Körperorganen und wird charakterisiert durch die Differenz des Sauerstoffgehalts im arteriellen und venösen Blut. Beim gesunden Menschen sinkt die gemischtvenöse Sättigung unter Belastung, wenn sich der Sauerstoffbedarf der Organe erhöht. Kompensatorisch wird gleichzeitig die Auswurfleistung des Herzens vermehrt (Anstieg der Herzfrequenz), um den Sauerstoffbedarf der Muskulatur und der inneren Organe sicherzustellen. Während der Behandlung eines Dialysepatienten wird keine körperliche Arbeit verrichtet, jedoch sinkt bei kritischem Flüssigkeitsentzug das effektive zirkulierende Blutvolumen ohne adäquaten Anstieg der Herzfrequenz, wodurch bei konstantem Sauerstoffbedarf der relative Sauerstoffentzug aus dem arteriellen Blut erhöht wird und die venöse Sauerstoffsättigung absinkt. Somit ist der Abfall der venösen Sauerstoffsättigung korreliert mit einer Reduktion der Auswurfleistung des Herzens (Fick'sches Prinzip). Eine dramatische Absenkung führt dann zu einer Sauerstoffunterversorgung des Gewebes, die möglicherweise ein Vorläufer für das Auftreten von Symptomen wie Blutdrückabfällen oder Krämpfen ist.

Bei einer arteriellen Sauerstoffsättigung von 92-100 % beträgt die gemischt-venöse Sättigung beim gesunden Menschen in Ruhe etwa 70% und dies ist auch bei Dialysepatienten die Regel, sofern nicht weitere Organschäden - wie ein fortgeschrittenes Herzversagen - vorliegen, die die Sauerstoffausschöpfung beeinflußen. Es wurde nun beobachtet, daß Symptome mit erhöhter Häufigkeit auftreten, wenn die im extrakorporalen Kreislauf gemessene Sauerstoffsättigung des mit Hilfe von zentralvenösen Kathetern entnommenen Blutes auf 30% oder darunter abfällt. Symptome konnten in der Folge dadurch verhindert werden, daß das Absinken der Sauerstöffsättigung beobachtet und noch vor dem Erreichen von 30% die Ultrafiltrationsrate gedrosselt wurde.

Es wurde ferner beobachtet, daß bei Patienten mit Herzinsuffizienz die Sauerstoffsättigung zu Beginn der Behandlung unterhalb von 70% lag und sich im Laufe der Behandlung zunächst bis auf Nahe an 70% erhöhte um dann wieder abzufallen.

Dementsprechend wird vorgeschlagen, die Ultrafiltrationseinrichtung eines Hämodialyse-, Hämofiltrations- oder Hämodiafiltrationsgerätes in Abhängigkeit von der Sauerstoffsättigung des extrakorporalen Blutes zu steuern. Dies kann im einfachsten Fall so erfolgen, daß das Sauerstoffsättigungsmeßgerät mit einer einstellbaren Alarmschwelle ausgestattet ist und bei Absinken der Sauerstoffsättigung unter den eingestellten Grenzwert ein Signal ausgelöst wird, das den Bediener des Hämodialysegerätes zum Abschalten der Ultrafiltration veranlaßt. In einer weiteren Ausgestaltung kann diese Abschaltung automatisch erfolgen. Eine weitere Verbesserung ist durch eine proportionale Steuerung der Ultrafiltration in Abhängigkeit von der Abweichung der Sauerstoffsättigung vom Anfangswert möglich. Alternativ kann auch die Veränderungsgeschwindigkeit der Sauerstoffsättigung allein oder in Kombination mit einer der zuvor genannten Maßnahmen zur Steuerung herangezogen werden. Die Steuerungssoftware kann dabei bekannte Regelalgorithmen, z.B. PD Steuerung oder fuzzy-logic benutzen.

Die Steuerung kann mit Hilfe einer der beschriebenen Regelalgorithmen auch so erfolgen, daß zunächst eine Ultrafiltrationsrate eingestellt wird die höher, als die aus Ultrafiltrationsvolumen durch Behandlungszeit errechnete Rate ist und in der Folge die Ultrafiltration unterbrochen oder reduziert wird, wenn die Sauerstoffsättigung abfällt oder wenn die vorgegebene Ultrafiltrationsmenge erreicht ist.

Das Blut muß dabei über einen doppellumigen zentralvenösen Katheter mit Positionierung der Spitzen im rechten Vorhof entnommen werden, kann aber alternativ zu einem doppellumigen Katheter nach Entnahme mittels eines einlumigen Katheters über einen anderen Weg in den Kreislauf zurückgegeben werden, z.B. über einen peripheren Shunt (Fistel) oder kurzen Katheter, der nicht in eine Zentralvene führt. Auf diese Weise kann beim ruhenden Patienten, der keine körperliche Arbeit verrichtet, eine kontinuierliche Kontrolle über die Veränderung der Herzauswurfleistung vorgenommen werden. Somit läßt sich auch bei Patienten mit kardialen Problemen ohne Dialyse-Pflichtigkeit der Einfluß von therapeutischen Maßnahmen (z. B. kreislaufunterstützende Behandlung mit Medikamenten) validieren. Dieses Verfahren stellt eine Alternative zur intermittierenden Kontrolle des Herzminutenvolumens durch Injektion von kalter Kochsalzlösung dar (Thermodilutionsverfahren) und bietet ohne zusätzlichen Personaleinsatz eine permanente Überwachung einer kritischen Größe zur Kontrolle der Hämodynamik,

Der überwiegende Teil des Sauerstoffs im Blut ist an Hämoglobin gebunden. Dieser Sauerstoff steht im Gleichgewicht mit dem physikalisch gelösten Sauerstoff im Plasma. Dieses Gleichgewicht wird durch die Sauerstoffsättigungskurve beschrieben, die die Sauerstoffsättigung des Hämoglobins gegen den Sauerstoffpartialdruck im Plasma beschreibt. Diese Kurve ist im Bereich in dem eine Intervention, d.h. eine Reduzierung oder Abschaltung der Ultrafiltration sinnvollerweise erfolgt, annähernd linear. Dialysatoren erlauben nicht nur den Austausch von festen, in Wasser gelösten Substanzen, sondern auch von gelösten Gasen. Es wurde festgestellt, daß der Sauerstoffpartialdruck im verbrauchten Dialysat mit der Sauerstoffsättigung im Blut korreliert. Das Verfahren zur Steuerung der Ultrafiltration auf der Basis der Sauerstoffsättigung kann daher auch mit Hilfe eines Sauerstoffpartialdrucksensors im verbrauchten Dialysat erfolgen. Bei der Hämofiltration wird der Sauerstoffpartialdruck direkt im Filtrat gemessen. Da dies anaerob erfolgt entspricht der Sauerstoffpartialdruck direkt dem des Plasmas wie dies in der DE3616062 beschrieben ist. Bei der Hämodialyse enthält das Dialysat, abhängig von der Qualität der Entgasung, bereits eine unbekannte Menge an Sauerstoff. Erforderlichenfalls kann man daher einen zusätzlichen Sauerstoffsensor stromauf des Dialysators im Dialysatkreislauf anordnen und aus der Differenz der Partialdrücke sowie der Dialysatorclearance für Sauerstoff auf den Plasmasauerstoffgehalt schließen. Die Dialysatorclearance kann aus der, in der Regel bekannten Clearance für Harnstoff in bekannter Weise errechnet werden. Dazu wird der Massentransferkoeffizient für Sauerstoff aus dem Massentransferkoeffizienten für Harnstoff entsprechend dem Verhältnis der tabellierten Diffusionskonstanten berechnet und dann aus dem Massentransferkoeffizienten in bekannter Weise die Clearance. Auch kann an Stelle der tabellierten Clearancewerte für Harnstoff in bekannter Weise eine Messung vorgenommen werden, wie sie z.B. in der DE3938662 beschrieben ist.

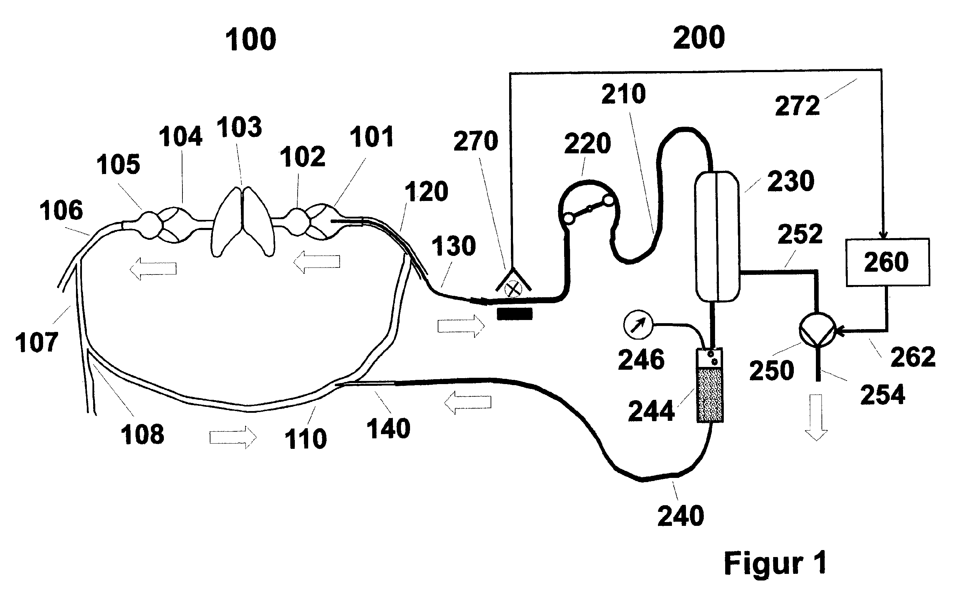

Eine Ausgestaltung der Erfindung ist in Figur 1 dargestellt. 100 stellt symbolisch den physiologischen Blutkreislauf und 200 den extrakorporalen Kreislauf dar.

Blut fließt im natürlichen Blutkreislauf vom Vorhof des rechten Herzens 101 zur rechten Herzkammer 102 und wird durch diese zur Lunge 103 gepumpt. Von dort gelangt es in die linke Vorkammer 104 und den linken Ventrikel 105 und weiter in die Aorta 106 von der dann weitere Arterien abzweigen von denen lediglich eine (107) dargestellt ist. Dabei handelt es sich z.B. um eine Armarterie, die an der Anastomose 108 mit einer Armvene 110 verbunden ist, wodurch eine Arterio-venöse Fistel gebildet wird, die mit Hilfe einer Kanüle punktiert werden kann. Die Vene 110 mündet in die Zentralvene (vena cava) 120 die zurück zum rechten Vorhof 101 führt.

Über den zentralvenösen Katheter 130 der über eine periphere Vene in die Zentralvene geschoben und so plaziert wird, daß die Spitze im rechten Vorhof 101 zu liegen kommt wird Blut durch die Blutpumpe 220 entnommen. Der Katheter 130 ist mit dem arteriellen Blutschlauchsystem 210 verbunden, das am anderen Ende mit dem Hämodialysator, Hämofilter oder Hämodiafilter 230 verbunden ist. Dieser wird durch eine nicht näher bezeichnete Membran in ein Blut- und ein Dialysat oder Filtratteil geteilt. Dazwischen liegt die peristaltische Blutpumpe 220. Vom Hämodialysator 230 führt das venöse Schlauchsystem 240 zur Kanüle 140, die in die Fistel 110 eingeführt ist. Alternativ kann das venöse Schlauchsystem mit einem weitere Katheter verbunden sein, der in einer geeigneten Vene plaziert ist. Auch kann das venöse Schlauchsystem mit dem venösen Schenkel eines Dopplelumenkatheters verbunden sein. Im venösen Schlauchsystem ist eine Tropfkammer 244 eingefügt, die mit einem Drucksensor 246 verbunden ist. Vom Filtratteil des Dialysator/Hämofilter/Hämodiafilter zweigt eine Leitung 252 zu einer Pumpe 250 ab, mit Hilfe welcher kontrolliert Flüssigkeit durch Ultrafiltration entzogen werden kann. Komponenten zur Durchleitung von Dialysat sind nicht dargestellt, da sie dem Fachmann geläufig sind. Die Ultrafiltratpumpe 250 fördert entzogene Flüssigkeit über die Leitung 254 zum Abfluß. Die Pumpe 250 wird von der Steuereinheit 260, die über eine elektrische Leitung 262 mit der Pumpe 250 verbunden ist, gesteuert.

Am arteriellen Blutschlauchsystem ist der optische Sensor 270 zur Messung der Sauerstoffsättigung angebracht. Dieser ist mit der Ultrafiltrationssteuereinheit über die Leitung 272 verbunden. Der Sensor 270 kann sowohl im arteriellen (210) als auch im venösen (240) Teil des Schlauchsystems angeordnet werden, da die Sauerstoffsättigung des Blutes durch die Hämofiltration oder Hämodialyse nur geringfügig verändert wird. Bevorzugt ist die Position im arteriellen Bereich, da dabei die Verzögerungszeit etwas geringer ist. Wird der Sensor im venösen Bereich angeordnet, so kann er mit einer Einrichtung zur Erkennung von Blut, Gas oder Flüssigkeit kombiniert werden, wie sie u.a. in EPA 0467805 beschrieben ist. Auch ist eine Kombination mit Sensoren für andere Parameter möglich, die optisch erfaßt werden können. Dazu gehören z.B. Harnstoff oder Glucose, die im Infrarotbereich absorbieren.

Die Steuerung der Ultrafiltratpumpe 250 durch die Steuereinheit 260 in Abhängigkeit vom Signal des Sensors 270 kann wie weiter oben beschrieben erfolgen. Zur Wahl des Steuerprogramms sowie der Steuerparameter verfügt die Steuereinheit über entsprechende Eingabeelemente. Dem Stand der Technik entsprechend können die der Steuereinheit 260 zugeordneten Anzeige- und Steuerelemente durch Mikroprozessoren und Bildschirme verwirklicht werden.

## Patentansprüche

1. Verfahren zur Ultrafiltrationskontrolle bei der Hämodialyse, Hämofiltration oder Hämodiafiltration mit einem rechtsatrialen Katheter zur Blutentnahme, einem extrakorporalen Kreislauf und einem Dialysat- oder Filtratteil, einer Einrichtung zum Entzug von Flüssigkeit mit einer Steuereinheit sowie wenigstens einem Sensor zur Messung des Sauerstoffgehalts im Blut oder Dialysat bzw. Filtrat **dadurch gekennzeichnet, daß** die Einrichtung zum Entzug von Flüssigkeit in Abhängigkeit vom Sauerstoffgehalt im Blut oder verbrauchten Dialysat bzw. Filtrat gesteuert wird.

2. Verfahren nach Anspruch 1, **dadurch gekennzeichnet, daß** der Sensor zur Messung des Sauerstoffgehalts die Sauerstoffsättigung des Blutes im extrakorporalen Kreislauf mißt.

3. Verfahren nach Anspruch 1, **dadurch gekennzeichnet, daß** der Sensor zur Messung des Sauerstoffgehalts den Sauerstoffpartialdruck im Blut des extrakorporalen Kreislaufs mißt.

4. Verfahren nach Anspruch 1, **dadurch gekennzeichnet, daß** der Sensor zur Messung des Sauerstoffgehalts den Sauerstoffpartialdruck im verbrauchten Dialysat bzw. Filtrat mißt.

5. Verfahren nach einem der vorhergegangenen Ansprüche, **dadurch gekennzeichnet, daß** zusätzlich zur Sauerstoffsättigung weitere Parameter zur Steuerung der Ultrafiltrationskontrolle herangezogen werden.

6. Verfahren nach Anspruch 5, **dadurch gekennzeichnet, daß** der zusätzliche Parameter die Veränderung des Blutvolumens ist.

7. Verfahren nach Anspruch 5, **dadurch gekennzeichnet, daß** der zusätzliche Parameter der Blutdruck ist.

8. Verfahren nach Anspruch 5, **dadurch gekennzeichnet, daß** der zusätzliche Parameter die Körpertemperatur oder Bluttemperatur ist.

9. Verfahren nach Anspruch 5, **dadurch gekennzeichnet, daß** der zusätzliche Parameter die Veränderung der Plasmaelektrolytkonzentration ist.

10. Vorrichtung zur Steuerung der Ultrafiltrationskontrolle bei der Hämodialyse, Hämofiltration oder Hämodiafiltration mit mindestens einem rechtsarteriellem Katheter, einem extrakorporalen Kreislauf mit mindestems einem Hämodialysator, Hämofilter oder Hämodiafilter, einem Dialysierflüssigkeits oder Filtratkreislauf, einem Sensor zur Messung des Sauerstoffgehalts sowie einer Einrichtung zum kontrollierten Entzug von Flüssigkeit und einer dazugehörigen Steuereinheit, **dadurch gekennzeichnet, daß** die Steuereinheit mit dem Sauerstoffsensor verbunden und der Flüssigkeitsentzug in Abhängigkeit vom Signal des Sauerstoffensors steuerbar ist.
